# EUROPÄISCHE PATENTSCHRIFT

(11) **EP 0 912 147 B1**
(45) Veröffentlichungstag und Bekanntmachung des Hinweises auf die Patenterteilung: **04.06.2003**
(21) Anmeldenummer: 97939993.8
(22) Anmeldetag: 12.07.1997
(51) Int. Cl.: A61F 2/44

(54) **IMPLANTAT ZUR WIRBELKÖRPERFUSION**
IMPLANT FOR VERTEBRAL BODY FUSION
IMPLANT POUR FUSION DE CORPS VERTEBRAUX

(30) Priorität: 15.07.1996 DE 19628473
(43) Veröffentlichungstag der Anmeldung: 06.05.1999
(73) Patentinhaber: Aesculap AG & Co. KG, 78532 Tuttlingen (DE)
(72) Erfinder: WING, Charles, D-78194 Immendingen (DE); EISEN, Guntmar, D-78532 Tuttlingen (DE); SCHULTZ, Robert, D-78532 Tuttlingen (DE)
(74) Vertreter: Böhme, Ulrich, Dr. Dipl.-Phys.
(86) Internationale Anmeldenummer: EP9703730
(87) Internationale Veröffentlichungsnummer: WO98002117

(56) Entgegenhaltungen:
- EP-A- 0 664 994
- WO-A-97/15246
- DE-U- 29 612 269

## Beschreibung

Die Erfindung betrifft ein Implantat zur Wirbelkörperfusion mit einem zwei gegenüberliegende Stützflächen zur Anlage an den einander zugewandten Endflächen der zu fusionierenden Wirbelkörper aufweisenden, in den Spalt zwischen den beiden benachbarten Wirbelkörpern einschiebbaren Stützkörper.

Bei der Beschädigung von Bandscheiben ist es häufig notwendig, diese zu entfernen und die benachbarten Wirbelkörper dauerhaft miteinander zu verbinden. Um dies zu erreichen, ist es bekannt, anstelle der entfernten Bandscheibe einen Stützkörper in den Zwischenwirbelraum einzuschieben, der im wesentlichen quaderförmig oder keilförmig ausgebildet ist und dort die Stütz- und Abstandsfunktion der Bandscheibe übernehmen soll (WO 95/08964).

Um hier eine einigermaßen sichere Fixierung des Stützkörpers im Zwischenwirbelraum zu erreichen, muß dieser stark profiliert werden, und dies erschwert das Einführen.

Andererseits ist es auch bekannt, in den Zwischenwirbelraum seitlich eine hohlzylinderförmige Knochenschraube einzudrehen, die dann in halbschalenförmige Innengewindebereiche der beiden Wirbelkörper eingreift und diese so gegeneinander festlegt. Eine solche Knochenschraube ist gegen axiale Verschiebung gesichert, jedoch ergeben sich aufgrund des relativ geringen Flächenkontaktes hohe Druckbelastungen, die teilweise dazu führen können, daß das knochenschraubenförmige Implantat in den Wirbelkörper einsinkt (EP 0369603 A1).

In der nicht vorveröffentlichten WO 97/15246 ist außerdem ein zweiteiliges Implantat mit einem Käfig und einem Rotationskörper beschrieben, der Rotationskörper ist dauerhaft drehbar in dem Käfig gelagert und kann beim Einschrauben in einen Zwischenwirbelraum das gesamte Implantat in den Zwischenwirbelraum hineinziehen.

Es ist Aufgabe der Erfindung, ein Implantat zu schaffen, das einerseits leicht einsetzbar ist und das andererseits nach dem Einsetzen eine Fixierung gegen Verschiebung erfährt.

Diese Aufgabe wird bei einem Implantat der eingangs beschriebenen Art erfindungsgemäß dadurch gelöst, daß der Stützkörper mindestens einen zwischen den Stützflächen verlaufenden Aufnahmekanal aufweist, der an seiner Oberseite und an seiner Unterseite offen ist, und daß in den Aufnahmekanal durch eine mit diesem fluchtende Einführöffnung hindurch ein mit einem außenseitigen Vorsprung versehenes, in dem Aufnahmekanal um seine Längsachse drehbar geführtes Fixierungselement einführbar ist, das im in den Aufnahmekanal eingeführten Zustand an der Oberseite und an der Unterseite des Stützkörpers über die Stützflächen hervorragend aus dem offenen Aufnahmekanal herausragt.

Es wird also ein zweiteiliges Implantat vorgeschlagen, das im wesentlichen einen Stützkörper aufweist, der mit seinen Stützflächen an den Endflächen der Wirbelkörper anliegt und dadurch diese Wirbelkörper großflächig gegeneinander abstützt. Zusätzlich wird ein Fixierungselement vorgesehen, das nach oben und nach unten über diesen Stützkörper hervorsteht und damit in entsprechend halbschalenförmige Ausnehmungen der Wirbelkörper formschlüssig eingreift.

Einen wesentlichen Teil der Abstützkräfte übernimmt bei dieser Konstruktion der Stützkörper, das Fixierungselement trägt in eingeschränktem Maße auch dazu bei, wesentlich ist jedoch, daß das Fixierungselement durch den Formschluß mit den beiden Wirbelkörpern gegenüber diesen Wirbelkörpern unverschiebbar ist. Durch die Aufnahme des Fixierungselementes in dem Aufnahmekanal wird auch der Stützkörper gegenüber den Wirbelkörpern festgelegt.

Während es grundsätzlich möglich ist, daß das Fixierungselement in dem Aufnahmekanal nur in Querrichtung geführt und dadurch festgelegt wird, sind bei einer bevorzugten Ausführungsform Mittel vorgesehen, die das in den Aufnahmekanal eingeführte Fixierungselement in diesem in axialer Richtung festlegen. Diese Mittel verbinden also das Fixierungselement und den Stützkörper auch in axialer Richtung, und dadurch wird auch der Stützkörper in axialer Richtung unverschiebbar gegenüber den Wirbelkörpern festgelegt.

Das Fixierungselement ist im Aufnahmekanal drehbar gelagert, der Formschluß mit den Wirbelkörpern wird durch Verdrehung des Fixierungselementes erzeugt. Dabei könnte das Fixierungselement beispielsweise nach Art eines Schlüssels einen bartförmigen Vorsprung tragen, der in einer Winkelstellung des Fixierungselementes nicht über die Stützflächen hervorsteht, so daß der Stützkörper in den Zwischenwirbelraum eingeschoben werden kann, während diese Vorsprünge durch Verdrehung des Fixierungselementes über die Stützflächen angehoben werden und dabei formschlüssig in die benachbarten Wirbelkörper eingreifen. Man erhält bei einem solchen Fixierungselement eine schlüsselartige Verriegelung beim Verdrehen des Fixierungselementes.

Gemäß einer anderen bevorzugten Ausführungsform ist vorgesehen, daß das Fixierungselement als Knochenschraube ausgebildet ist.

Eine solche Knochenschraube kann in unterschiedlicher Weise gegenüber dem Stützkörper festgelegt werden, beispielsweise könnte ein die Knochenschraube und die Stützkörper durchsetzender Stift eingesetzt werden, nachdem die Knochenschraube eingeschraubt ist.

Besonders vorteilhaft ist es, wenn gemäß einer bevorzugten Ausführungsform der Erfindung vorgesehen wird, daß die Mittel zur Festlegung der Knochenschraube in axialer Richtung ein mit dem Außengewinde der Knochenschraube zusammenpassendes Innengewinde im Aufnahmekanal umfassen. Bei einer solchen Lösung wird also die Knochenschraube in den Aufnahmekanal eingeschraubt, und gleichzeitig erfolgt ein Einschrauben in die benachbarten Wirbelkörper, wobei in diesen ein vorgefertigtes Innengewinde verwendet werden kann. Grundsätzlich wäre es aber auch möglich, die Knochenschraube selbstschneidend auszubilden, so daß sie sich ein entsprechendes Gewinde im Wirbelkörper selbst herstellt.

Bei einer bevorzugten Ausführungsform wird vorgesehen, daß der Aufnahmekanal die zu beiden Seiten desselben angeordneten Teile des Stützkörpers voneinander trennt und daß diese Teile durch eine Brücke miteinander verbunden sind.

Beispielsweise kann diese Brücke ein die beiden Teile verbindender Steg sein, der dem einschubseitigen Ende des Aufnahmekanals gegenüberliegt.

Bei einer anderen Ausführungsform kann vorgesehen sein, daß die Brücke am einschubseitigen Ende des Aufnahmekanals angeordnet ist und eine Durchstecköffnung für das Fixierungselement aufweist. Günstig ist es, wenn sich der Aufnahmekanal im wesentlichen über die gesamte Tiefe des Stützkörpers erstreckt, dadurch erhält man die Möglichkeit, ein langes Fixierungselement einzusetzen, das über einen großen Bereich dieser Länge mit den benachbarten Wirbelkörpern in Eingriff steht.

Grundsätzlich ist es möglich, nur einen einzigen Aufnahmekanal in einem Stützkörper vorzusehen, vorzugsweise in dessen Mitte, es wäre aber grundsätzlich auch möglich, daß in einem Stützkörper nebeneinander mehrere Aufnahmekanäle angeordnet sind, beispielsweise symmetrisch zur Mittellinie zwei derartige Aufnahmekanäle, die jeweils ein entsprechendes Fixierungselement aufnehmen.

Die Stützflächen können parallel zueinander ausgeführt sein, es ist aber grundsätzlich auch möglich, daß diese gegeneinander geneigt sind, so daß die Stützflächen an die Geometrie der Endflächen der Wirbelkörper angepaßt werden, insbesondere wenn es notwendig wird, die Wirbelkörper in bestimmter Weise gegeneinander zu neigen.

Bei einer bevorzugten Ausführungsform ist vorgesehen, daß die Stützflächen profiliert sind, beispielsweise können die Stützflächen quer zur Längsrichtung des Aufnahmekanals verlaufende Rippen tragen. Dies trägt zur Fixierung der Stützkörper im Zwischenwirbelraum erheblich bei und sichert eine dauerhafte, innige Verbindung zwischen Knochengewebe und Stützfläche.

Dabei ist es besonders vorteilhaft, wenn die Stützflächen mit einer knochenfreundlichen Oberflächenbeschichtung versehen sind, die ein Einwachsen des Knochenmaterials begünstigen.

Es können zu diesem Zweck in den Stützflächen auch Vertiefungen für das Einwachsen von Knochengewebe angeordnet sein.

In einer besonders einfachen Ausgestaltung ist der Stützkörper im wesentlichen quaderförmig geformt.

Die Knochenschrauben können grundsätzlich beliebig ausgebildet sein, es ist aber besonders vorteilhaft, wenn die Knochenschrauben hohl sind und in ihrem Mantel Durchbrechungen aufweisen. Derartige Knochenschrauben können mit Knochenmaterial gefüllt werden und ermöglichen durch die Durchbrechungen hindurch ein Zusammenwachsen der Knochenfüllung im Inneren und der Wirbelkörper auf der Außenseite der Knochenschrauben. Dadurch kann eine knöcherne Brücke zwischen den Wirbelkörpern durch die Knochenschraube hindurchwachsen, so daß ein Implantat dieser Art einmal durch den Stützkörper eine optimale großflächige Abstützung bereitstellt, andererseits durch die knöcherne Brücke im Bereich der Knochenschraube auch eine biologische Vereinigung der Wirbelkörper erzeugt.

Günstig ist es dabei, wenn die Durchbrechungen durch längliche axiale Ausschnitte in der Wand der Knochenschraube entstehen, die die Wand nur im Bereich der Täler des Außengewindes durchbrechen. Der Innenquerschnitt der Knochenschraube kann bei einer bevorzugten Ausführungsform einen koaxialen, kreisförmigen Bereich aufweisen, an den sich auf gegenüberliegenden Seiten zwei parallele, streifenförmige, tangential zum kreisförmigen Bereich verlaufende Abschnitte anschließen. Diese streifenförmigen Bereiche können seitlich über den kreisförmigen Bereich überstehen. Dabei ist es günstig, wenn die streifenförmigen Bereiche an ihrem Ende im wesentlichen halbkreisförmig abschließen. Es hat sich herausgestellt, daß dadurch die Knochenschraube in geringem Umfange flexibel bleibt, ohne bei der Biegebelastung mechanisch beschädigt zu werden. Dies ist ausserordentlich wichtig, da durch eine geringe Flexibilität der Knochenschraube das durchwachsende Knochenmaterial mechanisch beansprucht wird, und diese mechanische Beanspruchung fördert das Knochenwachstum.

Bei einer anderen bevorzugten Ausführungsform der Erfindung ist vorgesehen, daß die Knochenschraube einen Kern aufweist, dessen Außendurchmesser kleiner ist als der Abstand der Wände des Aufnahmekanals, so daß zwischen diesen Wänden und dem Kern ein Spalt gebildet wird, den die Gewindegänge der Knochenschraube durchsetzen. Dabei ist der Kern insbesondere massiv ausgebildet.

Bei einer solchen Ausgestaltung kann der Spalt zwischen der Wand des Aufnahmekanals einerseits und dem Kern andererseits mit Knochenmaterial ausgefüllt werden, das ein Zusammenwachsen der beiden benachbarten Wirbelkörper ermöglicht. Das Knochenmaterial wird dabei durch die Gewindegänge der Knochenschraube, die sich im Spalt befinden, festgelegt und abgestützt, so daß unerwünschte Verschiebungen des Knochenmaterials verhindert werden können.

Vorzugsweise bestehen der Stützkörper und / oder die Fixierungselemente aus Titan oder aus einer hochfesten Titanlegierung.

Grundsätzlich ist es aber auch möglich, diese Bauteile aus Kunststoff herzustellen, insbesondere aus karbonfaserverstärktem Kunststoff oder aus einem resorbierbaren Kunststoff.

Bei einer anderen Ausführungsform ist vorgesehen, daß der Stützkörper aus Knochen- oder Knochenersatzmaterial besteht. Dabei ist es vorteilhaft, wenn das Fixierungselement aus resorbierbarem Kunststoff gefertigt ist. Bei einer solchen Ausgestaltung wird der Stützkörper selbst Teil der knöchernen Brücke zwischen den beiden Wirbelkörpern, der von dem Fixierungselement eingenommene Raum wird vom Fixierungselement bei dessen Resorption allmählich freigegeben und wird dann ebenfalls knöchern durchwachsen.

Die nachfolgende Beschreibung bevorzugter Ausführungsformen dient im Zusammenhang mit der Zeichnung der näheren Erläuterung. Es zeigen:
- Figur 1:: eine Querschnittsansicht durch zwei Wirbelkörper mit einem eingesetzten Zwischenwirbel-implantat gemäß einer ersten bevorzugten Ausführungsform;
- Figur 2:: eine Draufsicht auf das Implantat der Figur 1, wobei eine Hälfte parallel zu einer Stützfläche aufgeschnitten ist;
- Figur 3:: eine Ansicht des Implantates der Figur 1 von der Einschubseite her und
- Figur 4:: eine Seitenansicht des Implantates der Figur 1;
- Figur 5:: eine Ansicht ähnlich Figur 2 bei einem abgewandelten Ausführungsbeispiel eines Implantates;
- Figur 6:: eine Ansicht ähnlich Figur 3 bei dem Implantat der Figur 5;
- Figur 7:: eine Ansicht ähnlich Figur 2 bei einem abgewandelten Ausführungsbeispiel eines Implantates mit einer Knochenschraube mit einem Kern kleinen Durchmessers und
- Figur 8:: eine Ansicht ähnlich Figur 3 bei dem Implantat der Figur 7.

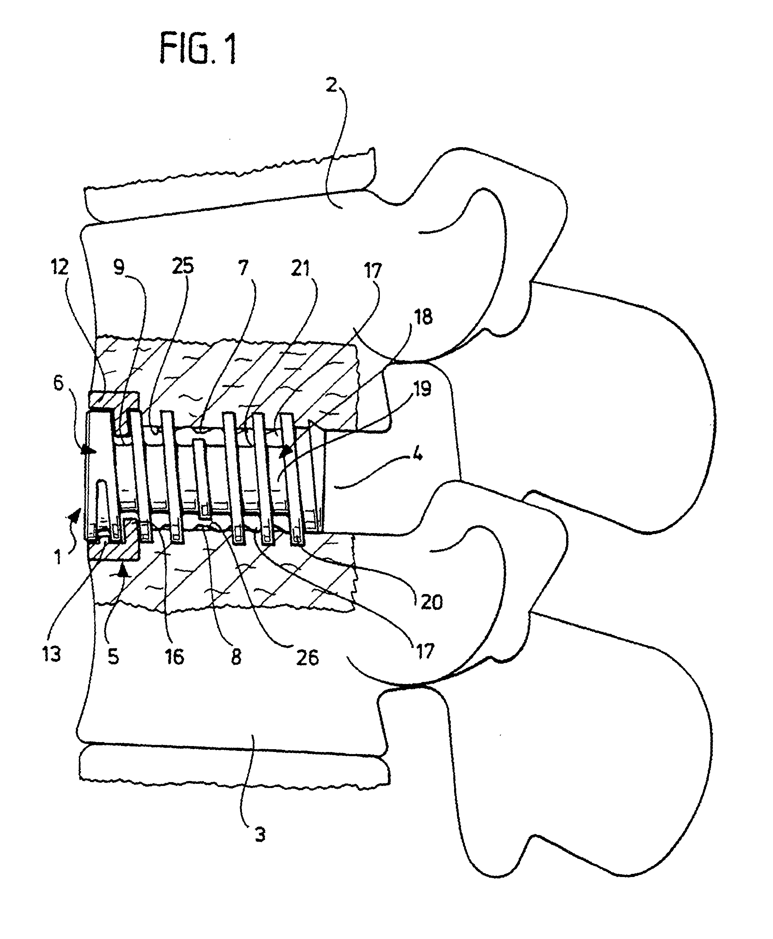

Das in der Zeichnung dargestellte Implantat 1 wird zwischen zwei Wirbelkörpern 2, 3 in den Zwischenwirbelraum 4 so eingesetzt, daß es die vorher entfernte Bandscheibe ersetzt und die benachbarten Wirbelkörper 2 und 3 zu einer Einheit verbindet, wie dies in Figur 1 dargestellt ist.

Das Implantat 1 umfaßt zwei Teile, nämlich einen im wesentlichen quaderförmigen Stützkörper 5 und eine in diesen eingesetzte Knochenschraube 6.

Der Stützkörper 5 weist an seiner oberen und seiner unteren Seite Stützflächen 7, 8 auf, die im wesentlichen eben und parallel zueinander ausgebildet sind, in seinen seitlichen Abmessungen erstreckt er sich über einen wesentlichen Teil des Zwischenwirbelraumes 4.

In der Mitte des Stützkörpers 5 weist dieser einen parallel zu den Stützflächen 7 und 8 verlaufenden, den Stützkörper 5 vollständig durchsetzenden Aufnahmekanal 9 mit einem kreiszylindrischen Querschnitt auf, der so bemessen ist, daß er an der Oberseite und an der Unterseite offen ist. Dadurch teilt dieser Aufnahmekanal 9 den Stützkörper 5 in zwei Teile 10, 11, die durch zwei bogenförmige Stege 12, 13 an der Oberseite und an der Unterseite des Stützkörpers 5 miteinander verbunden sind. Diese bogenförmigen Stege 12 und 13 bilden gemeinsam eine Öffnung 14a aus, die mit dem Aufnahmekanal 9 fluchtet.

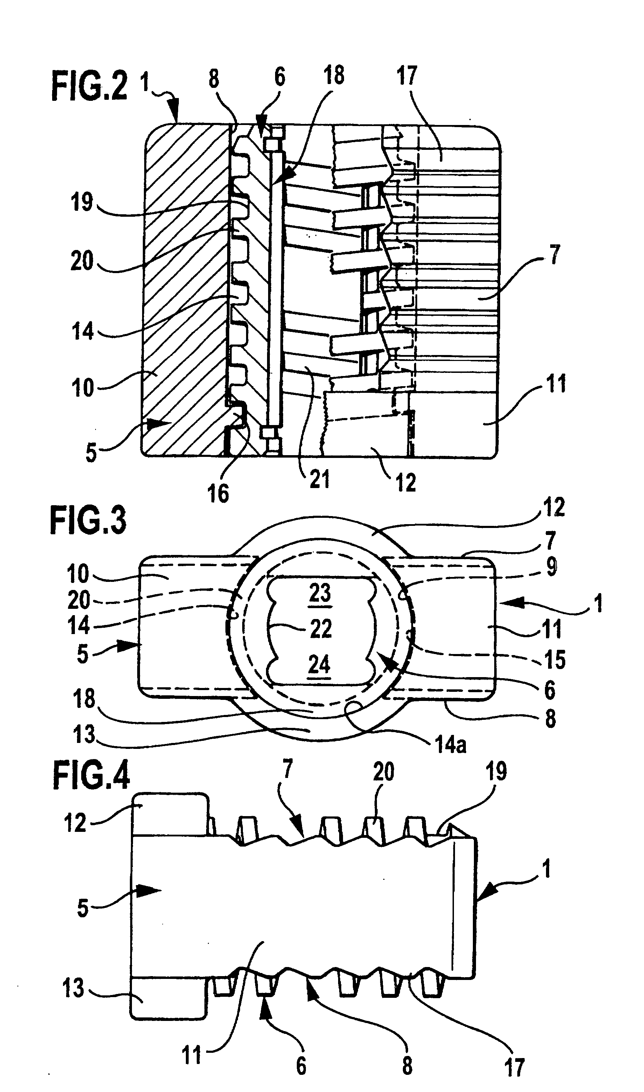

Im Inneren des Aufnahmekanals 9, der eigentlich nur durch zwei Innenwände 14, 15 der Teile 10 beziehungsweise 11 mit bogenförmigem Querschnitt ausgebildet wird, befindet sich ein Innengewinde, das im dargestellten Ausführungsbeispiel der Figuren 1 und 4 durch einen einzigen Gewindegang 16 im Einschubbereich des Aufnahmekanals 9 geformt ist.

Die Stützflächen 7 und 8 sind durch quer zur Längsrichtung des Aufnahmekanals 9 verlaufende Rippen 17 profiliert,
außerdem kann die Oberfläche mit einem knochenfreundlichen Material beschichtet sein, wie dies im Prothesen- und Implantatbau allgemein üblich ist.

Die in den Aufnahmekanal 9 eingesetzte Knochenschraube 6 ist ein zylindrischer Hohlkörper 18, auf dessen Aussenwand 19 ein durchgehender Gewindegang 20 aufgesetzt ist. Der Innenraum dieses Hohlkörpers 18 steht über Durchbrechungen 21 in der Außenwand 19 und dem Außenraum in Verbindung; die Durchbrechungen 21 sind allerdings auf den Bereich zwischen den Vorsprüngen des Gewindegangs 20 beschränkt. Die Querschnittsform des Innenraums des Hohlkörpers 18 läßt sich beschreiben durch einen kreisförmigen, koaxialen inneren Bereich 22 und durch an gegenüberliegenden Seiten tangential an diesen kreisförmigen Bereich 22 angesetzte streifenförmige Bereiche 23, 24, die parallel zueinander verlaufen, seitlich über den kreisförmigen Bereich 22 vorstehen und halbkreisförmig abgeschlossen sind (Figur 3). Es hat sich herausgestellt, daß eine solche Querschnittsform einerseits die Möglichkeit bietet, die Außenwand 19 des Hohlkörpers 18 bei den streifenförmigen Bereichen 23 und 24 zwischen den Gewindegängen 20 durch seitliche Einfräsungen zu durchbrechen, jedoch andererseits eine so hohe mechanische Stabilität beizubehalten, daß die Knochenschraube 6 trotz einer geringfügigen Flexibilität bei dieser mechanischen Verformung in keiner Weise beschädigt wird.

Der Gewindegang 20 hat einen Außendurchmesser, der dem Innendurchmesser der Innenwände 14 und 15 entspricht, so daß die Knochenschraube in dem durch die Innenwände 14 und 15 ausgebildeten Aufnahmekanal 9 geführt verdrehbar ist, wobei die Gewindegänge 20 und in geringem Ausmaß auch die Außenwand 19 über die Stützflächen 7 und 8 hervorstehen.

Zum Einsetzen des Implantates wird zunächst die beschädigte Bandscheibe entfernt, gegebenenfalls werden auch die Endflächen 25 beziehungsweise 26 der Wirbelkörper 2 und 3 durch geeignete Werkzeuge bearbeitet.

Anschließend wird der Stützkörper 5 in den Zwischenwirbelraum eingeschoben.

In den noch leeren Aufnahmekanal 9 kann ein Bohr- oder Fräswerkzeug eingeschoben werden, das in die Endflächen 25 und 26 der Wirbelkörper 2 beziehungsweise 3 halbschalenförmige Ausnehmungen einarbeitet, die zusammen mit den Innenwänden 14 und 15 des Aufnahmekanals 9 den Aufnahmekanal 9 bilden, wobei allerdings der Innendurchmesser dieser Halbschalen kleiner ist als der der Innenwände 14 und 15.

In diese halbschalenförmigen Ausnehmungen der Wirbelkörper 2 und 3 kann anschließend ein Gewinde geschnitten werden, das dem der Knochenschraube 6 entspricht.

Nach dieser Vorbereitung der Wirbelkörper wird die Knochenschraube 6 in den Aufnahmekanal 9 eingesetzt und eingeschraubt, wobei der Gewindegang 20 gleichzeitig in das Gewinde der Wirbelkörper 2 und 3 eingreift. Dadurch erfolgt eine Verriegelung des Stützkörpers 5 im Zwischenwirbelraum 4. Die Knochenschraube 6 wird mit Knochenmaterial gefüllt und bildet somit eine knöcherne Brücke zwischen den beiden Wirbelkörpern 2 und 3 aus, so daß durch die Durchbrechungen 21 in der Außenwand 19 das Knochenmaterial von den Wirbelkörpern in das Innere der Knochenschraube 6 vorwachsen kann.

Grundsätzlich steht es dem Chirurgen frei, den Stützkörper 5 auch ohne eine Knochenschraube 6 zu verwenden, falls er in bestimmten Fällen auf andere Weise eine ausreichende Fixierung des Stützkörpers 5 erreichen will.

Außerdem ist es auch möglich, die Knochenschraube 6 ohne den Stützkörper 5 zu implantieren, wie dies bei Knochenschrauben an sich bekannt ist.

Das beschriebene Implantat gibt dem Chirurgen also die Möglichkeit, wahlweise die besonders vorteilhaften Wirkungen des kombinierten Implantates auszunützen oder aber die beiden Teile des Implantates auch getrennt einzusetzen, wenn er dies für erforderlich hält.

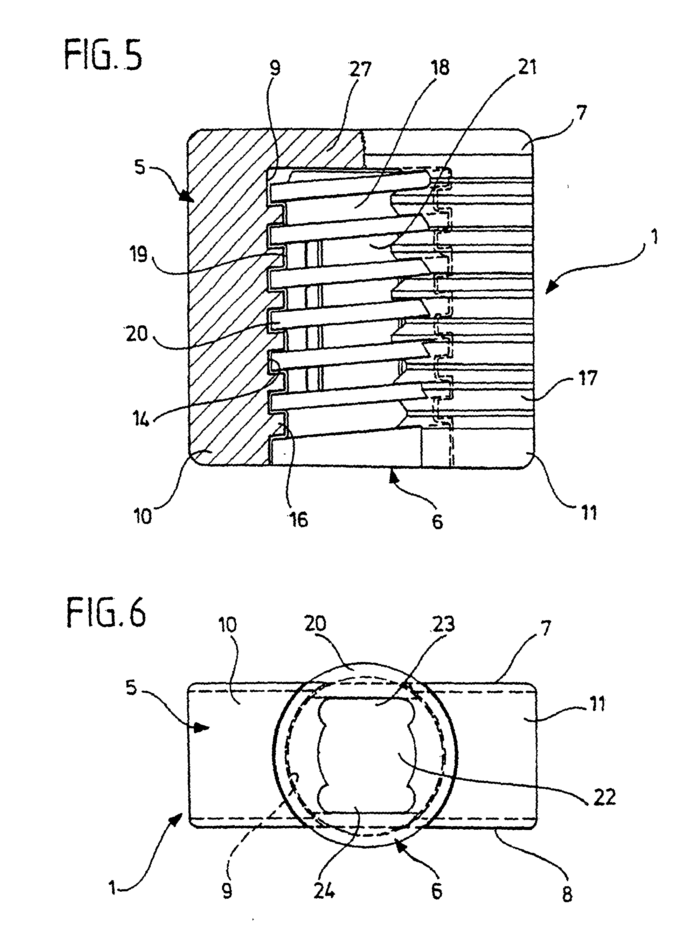

Das in den Figuren 5 und 6 dargestellte Implantat entspricht dem der Figuren 1 bis 4 weitgehend, einander entsprechende Teile sind daher mit denselben Bezugszeichen gekennzeichnet.

Im Unterschied zu dem Stützkörper 5 der Figuren 1 bis 4 sind die beiden Teile 10 und 11 hier nicht durch bogenförmige Stege 12 und 13 am einschubseitigen Ende des Aufnahmekanals 9 verbunden, sondern durch einen Steg 27 am gegenüberliegenden Ende des Aufnahmekanals 9. Dieser Steg 27 verschließt den Aufnahmekanal 9 an diesem Ende, so daß damit auch die Einschraubtiefe der Knochenschraube 6 begrenzt wird.

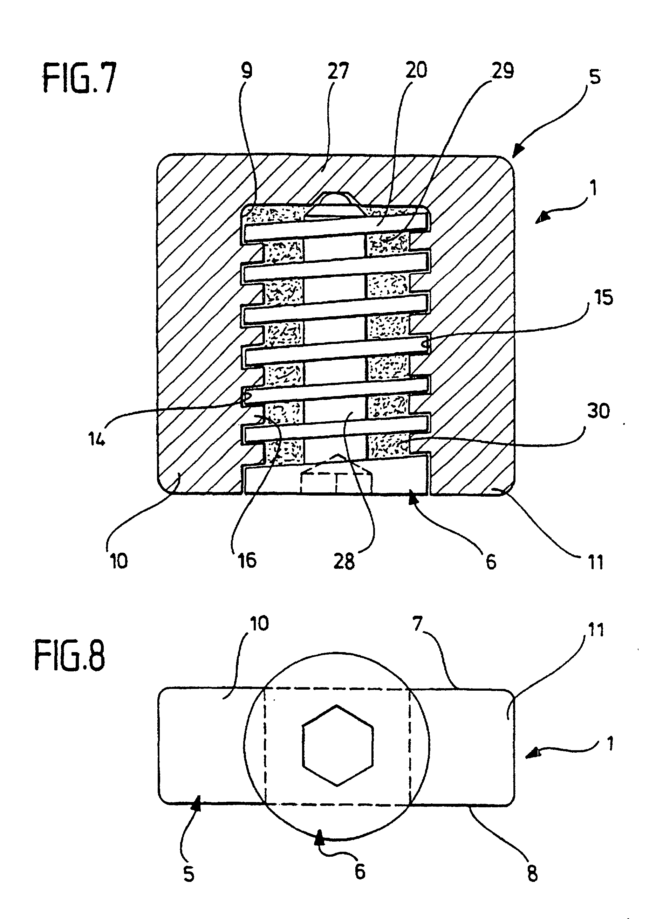

Bei dem Ausführungsbeispiel der Figuren 7 und 8, bei dem wieder entsprechende Teile dieselben Bezugszeichen tragen, ist die Knochenschraube 6 mit einem massiven Kern 28 versehen, der den Gewindegang 20 trägt. Der Außendurchmesser des Kerns 28 ist deutlich geringer als der Abstand der einander gegenüberliegenden Wände 14 und 15 des Aufnahmekanals 9, so daß zwischen dem Kern 28 und den Wänden 14, 15 ein Spalt 29 entsteht. Dieser Spalt 29 kann bei eingesetzter Knochenschraube mit Knochenmaterial 30 aufgefüllt werden, so daß dieses Knochenmaterial eine knöcherne Brücke zwischen den zu fusionierenden wirbelkörpern bildet. Das Knochenmaterial 30 wird dabei zwischen den benachbarten Gängen des Gewindegangs 20 festgelegt.

## Patentansprüche

1. Implantat zur Wirbelkörperfusion mit einem zwei gegenüberliegende Stützflächen zur Anlage an den einander zugewandten Endflächen der zu fusionierenden Wirbelkörper aufweisenden, in den Spalt zwischen den beiden benachbarten Wirbelkörpern einschiebbaren Stützkörper,
wobei der Stützkörper (5) mindestens einen zwischen den Stützflächen (7, 8) verlaufenden Aufnahmekanal (9) aufweist, der an seiner Oberseite und an seiner Unterseite offen ist, und daß in den Aufnahmekanal (9) durch eine mit dem Aufnahmekanal (9) fluchtende Einführöffnung (14a) hindurch ein mit einem außenseitigen Vorsprung (20) versehenes, in dem Aufnahmekanal (9) um seine Längsachse drehbar geführtes Fixierungselement (6) einführbar ist, das in in den Aufnahmekanal (9) eingeführtem Zustand an der Oberseite und an der Unterseite des Stützkörpers (5) über die Stützflächen (7, 8) hervorragend aus dem offenen Aufnahmekanal (9) hervorsteht.

2. Implantat nach Anspruch 1, **dadurch gekennzeichnet, daß** Mittel vorgesehen sind, die das in den Aufnahmekanal (9) eingeführte Fixierungselement (6) in diesem in axialer Richtung festlegen.

3. Implantat nach Anspruch 1 oder 2, **dadurch gekennzeichnet, daß** das Fixierungselement eine Knochenschraube (6) ist.

4. Implantat nach Anspruch 2 oder 3, **dadurch gekennzeichnet, daß** die Mittel zur Festlegung der Knochenschraube (6) in axialer Richtung ein mit dem AuBengewinde (20) der Knochenschraube (6) zusammenpassendes Innengewinde (16) im Aufnahmekanal (9) umfassen.

5. Implantat nach einein der voranstehenden Ansprüche, **dadurch gekennzeichnet, daß** der Aufnahmekanal (9) die zu beiden Seiten desselben angeordneten Teile (10, 11) des Stützkörpers (5) voneinander trennt und daß diese Teile (10, 11) durch eine Brücke (12, 13; 27) miteinander verbunden sind.

6. Implantat nach Anspruch 5, **dadurch gekennzeichnet, daß** die Brücke ein die beiden Teile (10, 11) verbindender Steg (27) ist, der dem einschubseitigen Ende des Aufnahmekanals (9) gegenüberliegt.

7. Implantat nach Anspruch 6, **dadurch gekennzeichnet, daß** der Steg (27) den Aufnahmekanal (9) an dem der Einschubseite abgewandten Ende verschließt.

8. Implantat nach Anspruch 5, **dadurch gekennzeichnet, daß** die Brücke (12, 13) am einschubseitigen Ende des Aufnahmekanals (9) angeordnet ist und eine Durchstecköffnung für das Fixierungselement (6) aufweist.

9. Implantat nach einem der voranstehenden Ansprüche, **dadurch gekennzeichnet, daß** sich der Aufnahmekanal (9) im wesentlichen über die gesamte Tiefe des Stützkörpers (5) erstreckt.

10. Implantat nach einem der voranstehenden Ansprüche, **dadurch gekennzeichnet, daß** in einem Stützkörper (5) nebeneinander mehrere Aufnahmekanäle (9) angeordnet sind.

11. Implantat nach einem der voranstehenden Ansprüche, **dadurch gekennzeichnet, daß** die Stützflächen (7, 8) gegeneinander geneigt sind.

12. Implantat nach einem der voranstehenden Ansprüche, **dadurch gekennzeichnet**, daB die Stützflächen (7, 8) profiliert sind.

13. Implantat nach Anspruch 12, **dadurch gekennzeichnet, daß** die Stützflächen (7, 8) quer zur Längsrichtung des Aufnahmekanals (9) verlaufende Rippen (17) tragen.

14. Implantat nach einem der voranstehenden Ansprüche, **dadurch gekennzeichnet, daß** die Stützflächen (7, 8) mit einer knochenfreundlichen Oberflächenbeschichtung versehen sind.

15. Implantat nach einem der voranstehenden Ansprüche, **dadurch gekennzeichnet, daß** in den Stützflächen (7, 8) Vertiefungen für das Einwachsen von Knochengewebe angeordnet sind.

16. Implantat nach einem der voranstehenden Ansprüche, **dadurch gekennzeichnet, daß** die Stützkörper (5) im wesentlichen quaderförmig geformt sind.

17. Implantat nach einem der Ansprüche 3 bis 16, **dadurch gekennzeichnet, daß** die Knochenschrauben (6) hohl sind und in ihrem Mantel Durchbrechungen (21) aufweisen.

18. Implantat nach Anspruch 17, **dadurch gekennzeichnet, daß** die Durchbrechungen (21) durch längliche axiale Ausschnitte in der Wand (19) der Knochenschraube (6) entstehen, die die Wand (19) nur im Bereich der Täler des Außengewindes (20) durchbrechen.

19. Implantat nach Anspruch 17 oder 18, **dadurch gekennzeichnet, daß** der Innenquerschnitt der Knochenschraube (6) einen koaxialen kreisförmigen Bereich (22) aufweist, an den sich auf gegenüberliegenden Seiten zwei parallele, streifenförmige, tangential zum kreisförmigen Bereich (22) verlaufende Abschnitte (23, 24) anschließen.

20. Implantat nach Anspruch 19, **dadurch gekennzeichnet, daß** die streifenförmigen Bereiche (23, 24) seitlich über den kreisförmigen Bereich (22) überstehen.

21. Implantat nach Anspruch 19 oder 20, **dadurch gekennzeichnet, daß** die streifenförmigen Bereiche (23, 24) an ihrem Ende im wesentlichen halbkreisförmig abschließen.

22. Implantat nach einem der Ansprüche 3 bis 16, **dadurch gekennzeichnet, daß** die Knochenschraube (6) einen Kern (28) aufweist, dessen Außendurchmesser kleiner ist als der Abstand der Wände (14, 15) des Aufnahmekanals (9), so daß zwischen diesen Wänden (14, 15) und dem Kern (28) ein Spalt (29) gebildet wird, den die Gewindegänge (20) der Knochenschraubbe (6) durchsetzen.

23. Implantat nach Anspruch 22, **dadurch gekennzeichnet, daß** der Kern (28) massiv ist.

24. Implantat nach einem der voranstehenden Ansprüche, **dadurch gekennzeichnet, daß** der Stützkörper (5) und / oder das Fixierungselement (6) aus Titan oder einer Titanlegierung bestehen.

25. Implantat nach einem der Ansprüche 1 bis 23, **dadurch gekennzeichnet, daß** der Stützkörper (5) und/oder das Fixierungselement (6) aus Kunststoff bestehen.

26. Implantat nach Anspruch 25, **dadurch gekennzeichnet, daß** der Stützkörper (5) und / oder das Fixierungselement (6) aus einem karbonfaserverstärkten Kunststoff bestehen.

27. Implantat nach Anspruch 25, **dadurch gekennzeichnet, daß** der Stützkörper (5) und / oder das Fixierungselement (6) aus einem resorbierbaren Kunststoff bestehen.

28. Implantat nach einem der Ansprüche 1 bis 23, **dadurch gekennzeichnet, daß** der Stützkörper (5) aus Knochen- oder Knochenersatzmaterial besteht.

29. Implantat nach Anspruch 28, **dadurch gekennzeichnet, daß** das Fixierungselement (6) aus resorbierbarem Kunststoff besteht.

## Claims

1. An implant for the purpose of vertebral-body fusion, with a supporting body insertable into the gap between the two adjacent vertebral bodies and having two opposing supporting surfaces for positioning against the mutually facing end surfaces of the vertebral bodies to be fused,
wherein the supporting body (5) has at least one accommodating channel (9) following a course between the supporting surfaces (7, 8), which channel is open top and bottom, and a fixing member (6), provided with an outside projection (20) and rotatably guided within the accommodating channel (9) about its longitudinal axis, is introducible into the accommodating channel (9) through an insertion opening (14a) aligned with the accommodating channel (9), which fixing member (6) - in the state after being introduced into the accommodating channel (9) - projects out of the open accommodating channel (9) at the top and bottom of the vertebral body (5), jutting out beyond the supporting surfaces (7,8).

2. An implant according to Claim 1, **characterised in that** means are provided which fasten the fixing member (6) introduced into the accommodating channel (9), in the axial direction therein.

3. An implant according to Claim 1 or 2, **characterised in that** the fixing member is a bone-screw (6).

4. An implant according to Claim 2 or 3, **characterised in that** the means for fastening the bone-screw (6) in an axial direction comprise an internal thread (16) within the accommodating channel (9) matching the external thread (20) of the bone-screw (6).

5. An implant according to one of the preceding claims, **characterised in that** the accommodating channel (9) separates from one another the parts (10, 11) of the supporting body (5) arranged on both sides thereof, and **in that** these parts (10, 11) are connected to one another by means of a bridge (12, 13; 27).

6. An implant according to Claim 5, **characterised in that** the bridge is a member (27) connecting the two parts (10, 11), which member is positioned opposite the insertion-side end of the accommodating channel (9).

7. An implant according to Claim 6, **characterised in that** the member (27) closes the accommodating channel (9) at the end facing away from the insertion side.

8. An implant according to Claim 5, **characterised in that** the bridge (12, 13) is positioned on the insertion-side end of the accommodating channel (9) and has a through-opening for the fixing member (6).

9. An implant according to one of the preceding claims, **characterised in that** the accommodating channel (9) extends substantially over the whole depth of the supporting body (5).

10. An implant according to one of the preceding claims, **characterised in that** a plurality of accommodating channels (9) are arranged alongside one another in a supporting body (5).

11. An implant according to one of the preceding claims, **characterised in that** the supporting surfaces (7, 8) are inclined towards one another.

12. An implant according to one of the preceding claims, **characterised in that** the supporting surfaces (7, 8) are profiled.

13. An implant according to Claim 12, **characterised in that** the supporting surfaces (7, 8) bear ribs (17) following a course crosswise to the longitudinal direction of the accommodating channel (9).

14. An implant according to one of the preceding claims, **characterised in that** the supporting surfaces (7, 8) are provided with a bone-compatible surface coating.

15. An implant according to one of the preceding claims, **characterised in that** depressions are arranged in the supporting surfaces (7, 8) for the infiltration of bone tissue.

16. An implant according to one of the preceding claims, **characterised in that** the supporting bodies (5) are substantially parallelepipedal in shape.

17. An implant according to one of Claims 3 to 16, **characterised in that** the bone-screws (6) are hollow and have openings (21) in their sheathing.

18. An implant according to Claim 17, **characterised in that** the openings (21) arise through elongated axial cutouts in the wall (19) of the bone-screw (6), which penetrate through the wall (19) only in the region of the troughs of the external thread (20).

19. An implant according to Claim 17 or 18, **characterised in that** the internal cross-section of the bone-screw (6) has a coaxial circular region (22) adjoined on opposite sides by two parallel, strip-like portions (23, 24) following a course tangential to the circular region (22).

20. An implant according to Claim 19, **characterised in that** the strip-like regions (23, 24) project laterally over the circular region (22).

21. An implant according to Claim 19 or 20, **characterised in that** the strip-like regions (23, 24) terminate substantially in the form of a semi-circle at their end.

22. An implant according to one of Claims 3 to 16, **characterised in that** the bone-screw (6) has a core (28) the diameter of which is smaller than the distance of the walls (14, 15) of the accommodating channel (9), with the result that a gap (29) is formed between these walls (14, 15) and the core (28), which gap is penetrated by the thread turns (20) of the bone-screw (6).

23. An implant according to Claim 22, **characterised in that** the core (28) is solid.

24. An implant according to one of the preceding claims, **characterised in that** the supporting body (5) and/or the fixing member (6) are made of titanium or a titanium alloy.

25. An implant according to one of Claims 1 to 23, **characterised in that** the supporting body (5) and/or the fixing member (6) are made of a plastics material.

26. An implant according to Claim 25, **characterised in that** the supporting body (5) and/or the fixing member (6) are made of a carbon-fibre-reinforced plastics material.

27. An implant according to Claim 25, **characterised in that** supporting body (5) and/or the fixing member (6) are made of an absorbable plastics material.

28. An implant according to one of Claims 1 to 23, **characterised in that** the supporting body (5) is made of bone material or bone-substitute material.

29. An implant according to Claim 28, **characterised in that** fixing member (6) is made of absorbable plastics material.

## Revendications

1. Implant pour la fusion de corps vertébraux, comportant un corps de soutien présentant deux surfaces de soutien opposées, pour venir en appui sur les surfaces terminales tournées l'une vers l'autre des corps vertébraux à fusionner, pouvant être inséré entre les deux corps vertébraux voisins, le corps de soutien (5) présentant au moins un canal de réception (9) s'étendant entre les surfaces de soutien (7, 8), lequel est ouvert sur sa face supérieure et sur sa face inférieure, et un élément de fixation (6), guidé rotatif autour de son axe longitudinal dans le canal de réception (9) et pourvu d'une saillie (20) sur la face extérieure, pouvant être introduit dans le canal de réception (9) à travers une ouverture d'introduction (14a) en affleurement avec le canal de réception (9), lequel élément de fixation, à l'état d'introduction dans le canal de réception (9), fait saillie hors du canal de réception (9) ouvert sur la face supérieure et sur la face inférieure du corps de soutien (5) en dépassant les surfaces de soutien (7, 8).

2. Implant selon la revendication 1, **caractérisé en ce qu'**il est prévu des moyens qui immobilisent en direction axiale l'élément de fixation (6) introduit dans le canal de réception (9).

3. Implant selon l'une ou l'autre des revendications 1 et 2, **caractérisé en ce que** l'élément de fixation est une vis à os (6).

4. Implant selon l'une ou l'autre des revendications 2 et 3, **caractérisé en ce que** les moyens d'immobilisation de la vis à os (6) en direction axiale comprennent dans le canal de réception (9) un filet de vis intérieur (16) apparié au filet de vis extérieur (20) de la vis à os (6).

5. Implant selon l'une des revendications précédentes, **caractérisé en ce que** le canal de réception (9) sépare l'une de l'autre les parties (10, 11) du corps de soutien (5) agencées des deux côtés du canal de réception (9) et **en ce que** ces parties (10, 11) sont reliées l'une à l'autre par un pont (12, 13 ; 27).

6. Implant selon la revendication 5, **caractérisé en ce que** le pont est une traverse (27) reliant les deux parties (10, 11) qui est opposée à l'extrémité du canal de réception (9) située du côté d'insertion.

7. Implant selon la revendication 6, **caractérisé en ce que** la traverse (27) ferme le canal de réception (9) sur l'extrémité détournée du côté d'insertion.

8. Implant selon la revendication 5, **caractérisé en ce que** le pont (12, 13) est agencé à l'extrémité côté insertion du canal de réception (9) et présente une ouverture de passage pour l'élément de fixation (6).

9. Implant selon l'une des revendications précédentes, **caractérisé en ce que** le canal de réception (9) s'étend sensiblement sur toute la profondeur du corps de soutien (5).

10. Implant selon l'une des revendications précédentes, **caractérisé en ce que** dans un corps de soutien (5), plusieurs canaux de réception (9) sont agencés les uns à côté des autres.

11. Implant selon l'une des revendications précédentes, **caractérisé en ce que** les surfaces de soutien (7, 8) sont inclinées l'une contre l'autre.

12. Implant selon l'une des revendications précédentes, **caractérisé en ce que** les surfaces de soutien (7, 8) sont profilées.

13. Implant selon la revendication 12, **caractérisé en ce que** les surfaces de soutien (7, 8) portent des nervures (17) s'étendant transversalement à la direction longitudinale du canal de réception (9).

14. Implant selon l'une des revendications précédentes, **caractérisé en ce que** les surfaces de soutien (7, 8) sont pourvues d'un revêtement de surface toléré par les os.

15. Implant selon l'une des revendications précédentes, **caractérisé en ce que** dans les surfaces de soutien (7, 8) sont ménagés des renfoncements pour la croissance de tissu osseux.

16. Implant selon l'une des revendications précédentes, **caractérisé en ce que** les corps de soutien (5) sont formés sensiblement en forme de parallélépipède.

17. Implant selon l'une des revendications 3 à 16, **caractérisé en ce que** les vis à os (6) sont creuses et présentent des percées (21) dans leur enveloppe.

18. Implant selon la revendication 17, **caractérisé en ce que** les percées (21) sont formées par des découpes axiales allongées dans la paroi (19) de la vis à os (6) qui ne traversent la paroi (19) que dans la région des creux du filet de vis extérieur (20).

19. Implant selon l'une ou l'autre des revendications 17 et 18, **caractérisé en ce que** la section transversale intérieure de la vis à os (6) présente une région (22) circulaire coaxiale à laquelle se raccordent sur des côtés opposés deux tronçons (23, 24) parallèles en forme de bandes, s'étendant tangentiellement à la région circulaire (22).

20. Implant selon la revendication 19, **caractérisé en ce que** les tronçons (23, 24) en forme de bandes font saillie au-delà de la région circulaire (22).

21. Implant selon l'une ou l'autre des revendications 19 et 20, **caractérisé en ce que** les tronçons (23, 24) en forme de bandes se terminent sensiblement en forme de demi-cercle à leur extrémité.

22. Implant selon l'une des revendications 3 à 16, **caractérisé en ce que** la vis à os (6) présente un noyau (28) dont le diamètre extérieur est inférieur à la distance entre les parois (14, 15) du canal de réception (9) de sorte qu'entre ces parois (14, 15) et le noyau (28) est formée une fente (29) que traversent les filets de vis (20) de la vis à os (6).

23. Implant selon la revendication 22, **caractérisé en ce que** le noyau (28) est massif.

24. Implant selon l'une des revendications précédentes, **caractérisé en ce que** le corps de soutien (5) et/ou l'élément de fixation (6) est en titane ou en alliage de titane.

25. Implant selon l'une des revendications 1 à 23, **caractérisé en ce que** le corps de soutien (5) et/ou l'élément de fixation (6) sont en matière plastique.

26. Implant selon la revendication 25, **caractérisé en ce que** le corps de soutien (5) et/ou l'élément de fixation (6) sont en une matière plastique renforcée de fibres de carbone.

27. Implant selon la revendication 25, **caractérisé en ce que** le corps de soutien (5) et/ou l'élément de fixation (6) sont en une matière plastique résorbable.

28. Implant selon l'une des revendications 1 à 23, **caractérisé en ce que** le corps de soutien (5) est un matériau osseux ou de succédané osseux.

29. Implant selon la revendication 28, **caractérisé en ce que** l'élément de fixation (6) est en matière plastique résorbable.
